(19) **Europäisches Patentamt**

**European Patent Office**

**Office européen des brevets**

(11) Veröffentlichungsnummer : **0 015 229 B1**

(12) # EUROPÄISCHE PATENTSCHRIFT

(45) Veröffentlichungstag der Patentschrift :
05.10.83

(21) Anmeldenummer : 80730014.0

(22) Anmeldetag : 21.02.80

(51) Int. Cl.³ : **A 61 N   1/04**

---

(54) **Elektrode für einen künstlichen Herzschrittmacher.**

---

(30) Priorität : **21.02.79 DE 2907135**

(43) Veröffentlichungstag der Anmeldung :
**03.09.80 Patentblatt 80/18**

(45) Bekanntmachung des Hinweises auf die Patenterteilung : **05.10.83 Patentblatt 83/40**

(84) Benannte Vertragsstaaten :
**AT CH DE FR GB IT NL SE**

(56) Entgegenhaltungen :
**EP A 0 000 725**
**EP A 0 000 785**
**FR A 2 297 641**
**US A 3 416 534**
**US A 3 472 234**

**BIOMEDISCHE TECHNIK, Band 20, Nr. 1/2, 1. Februar 1979, Berlin, DE. H.J. BISPING : « Ubersicht über verankerbare transvenöse Schrittmachersonden », Selten 16-27**

(73) Patentinhaber : **BIOTRONIK Mess- und Theraplegeräte GmbH & Co Ingenleurbüro Berlin
Sleversufer 8
D-1000 Berlin 47 (DE)**

(72) Erfinder : **Karr, Dieter E. Dipl.-Phys.
Nehelmer Strasse 4
D-1000 Berlin 27 (DE)**
Erfinder : **Rlechert, Klaus-Dleter
Slemensstrasse 14
D-1000 Berlin 21 (DE)**

(74) Vertreter : **Christlansen, Henning, Dipl.-Ing.
Unter den Elchen 108a
D-1000 Berlin 45 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Elektrode für einen künstlichen Herzschrittmacher

Beschreibung

Die Erfindung betrifft eine Elektrode für einen künstlichen Herzschrittmacher für transvenöse Anwendung mit mindestens einem Befestigungselement, das an einem in einem mit einer Zuleitungswendel verbundenen Hohlzylinder geführten Kolben derart angeordnet ist, daß es zum Einführen der Elektrode soweit in den Hohlzylinder hineinschiebbar ist, daß es nicht mit dem Herzgewebe in Eingriff kommt, während es zum Fixieren der Elektrode durch eine mittels eines sich innerhalb der Zuleitungswendel erstreckenden Führungswerkzeugs ausgeübte Kraft über deren Frontfläche hinaus herausschiebbar ist.

Eine derartige Elektrode ist aus der DE-Al 21 33 304 bekannt. Bei dieser Elektrode befinden sich im Elektrodenkopf Drahthaken, die aus dem den Elektrodenkopf bildenden Hohlzylinder nach vorne austreten und, wenn der Kolben in der Endposition der Elektrode durch den über das Führungswerkzeug ausgübten Druck nach vorn geschoben wird, aufspringen und die Elektrode im Herzgewebe durch Verhaken fixieren.

Nachteilig bei dieser Art von Elektroden ist, daß die Drahthaken für den Elektrodenkopf nur eine schwache Verankerung gegenüber Kräften bilden, die in Richtung der Zuleitung, in herzabgewandter Orientierung angreifen. Außerdem fehlt eine hermetische Dichtung gegen eindringende Körperflüssigkeit zum Elektroden inneren hin.

Weiterhin sind aus der DE-Al 19 39 806 sowie der DE-Al 25 39 553 Elektroden bekannt, die an ihrem Ende eine schraubenförmige Wendel aufweisen, welche sich durch Ausübung eines Drehmomentes über die Zuleitung oder ein Führungswerkzeug in das Herzgewebe einschrauben lassen, wenn der Elektrodenkopf die vorgesehene Position im Herzen erreicht hat.

Diese Elektroden haben den Nachteil, daß sie zum transvenösen Einführen einen rohrförmigen, flexiblen Führungskatheter erfordern, da sich sonst, bevor die Elektrode ihre vorgesehene endgültige Position erreicht hat, die vorstehende korkenzieherförmige Wendel bereits mit dem Körpergewebe verhaken kann, wobei die Gefahr der Verletzung des Veneninneren oder der Herzklappen besteht.

Andererseits ist schon auf vielfältige Weise versucht worden, einen Schutz für die vorstehende Wendel einer Einschraubelektrode zu schaffen. In diesem Zusammenhang ist es aus der DE-Al 26 13 044 bekannt, die schraubenförmige Wendel während des Einführungsvorgangs durch eine diese seitlich umfassende elastische Manschette zu schützen. Diese Lösung ist jedoch unter anderem mit dem Nachteil eines im fixierten Zustand der Elektrode ständig ausgeübten Drucks der elastischen Manschette behaftet, der die Tendenz hat, die schraubenzieherförmige Wendel der Elektrodenspitze aus dem Herzgewebe zu lösen.

Bei der aus der DE-Al 25 33 766 bekannten Elektrode wird der Vorschub der Elektrodenspitze in bezug auf die elektrodenzuleitung durch eine durch die schraubenförmige Steigung der Wendel erzeugte Gewindewirkung ermöglicht. Eine derartige Lösung hat den Nachteil, daß das durch die Schraubenwendel gebildetes Gewinde eine relativ große Reibung erzeugt, da es gleichzeitig eine Dichtung des Elektroden inneren gegen eindringende Körperflüssigkeit bilden muß, so daß auch das zum Ausfahren des Befestigungselementes erforderliche Drehmoment, welches über die Elektrodenzuleitung übertragen werden muß, entsprechend groß ist. Ein derartiges Drehmoment bewirkt aber — insbesondere wenn zum Ausfahren eine größere Reibung überwunden werden muß — eine Verwindung der Elektrodenzuleitung, so daß nicht mehr gewährleistet ist, daß sich die Spitze der Elektrode nach dem Ausfahren des Befestigungselementes noch an der für die Fixierung vorgesehenen Stelle befindet.

Aus der Druckschrift Biomedizinische Technik, Band 20, Nr 1/2, 1 Februar 1979, ist eine Elektrode bekannt, die an ihrem Ende eine schraubenförmige Wendel aufweist, welche sich durch Ausübung eines Drehmoments über die Zuleitung einschrauben lässt.

Der Erfindung liegt die Aufgabe zugrunde, eine Elektrode der vorgenannten Gattung anzugeben, die die genannten Nachteile der bekannten Elektroden nicht aufweist, d. h. eine sichere Fixierung gewährleistet und gegen das Eindringen von Körperflüssigkeit gesichert ist sowie das Ausführen aller notwendigen Betätigungen bei kleinem Kraftaufwand vom herzfernen Ende der Elektrodenzuleitung der zuläßt. Sie soll darüberhinaus einfach und sicher zu handhaben sein, so daß der implantierende Arzt sich stets über die Stellung der Befestigungsmittel am Elektrodenkopf im klaren ist.

Diese Aufgabe wird erfindungsgemäß durch die in Anspruch 1 angegebenen Merkmale gelöst.

Besonders vorteilhaft ist bei der erfindungsgemäßen Elektrode neben der Möglichkeit der vereinfachten Einführung die Tatsache, daß zur Freigabe d. h. zum Ausfahren der Befestigungsmittel und zum Fixieren des Elektrodenendes durch den implantierenden Arzt vom herzfernen Elektrodenende her getrennte und leicht voneinander unterscheidbare Bewegungsvorgänge auszuführen sind. Diese Bewegungsvorgänge, d. h. das Vorschieben des Führungswerkzeugs (Mandrins) bzw. die Drehung desselben oder der Elektrodenzuleitung entsprechen in sinnvoller Weise den auszulösenden Funktionen, so daß hier eine klare Trennung gegeben ist und Verwechslungen bezüglich der Handhabung vermieden sind.

Wird — wie bei einer der beanspruchten Alternativen — das Drehmoment auf die Befestigungsmittel durch das Führungswerkzeug übertragen, so kann die Elektrodenzuleitung aus Gründen der Bruchsicherheit besonders weich ausgeführt sein, während im anderen Fall — wenn die Drehbewegung bei festgehaltenem Mandrin über die Zuleitung erzeugt wird dieser als verhältnismäßig steifer Draht mit den zum Einführen der Elektrode auf einem gewundenen Weg notwendige Krümmungen versehen sein kann. Zum Fixieren der Elektrode ist in diesem Fall kein Austausch gegen einen besonderen Mandrin notwendig, was sonst möglicherweise zu einem Verlust des einmal gefundenen Fixierungsortes der Elektrode führen kann.

Weiterhin ist bei der erfindungsgemäßen Elektrode vorteilhaft, daß durch die Dichtung des in dem Hohlzylinder gleitenden Kolbens ein Eindringen von Körperflüssigkeit in das Innere der Zuleitungswendel, die zum Durchlaß des Führungswerkzeugs hohl ausgeführt sein muß, verhindert ist. Eindringende Körperflüssigkeit könnte sonst durch Gerinnung zu einer Herabsetzung der Elastizität der Elektrodenzuleitung im Bereich des Kopfes führen. Trotz der erzielten Dichtigkeit ist der Kraftaufwand zum Ausfahren des Befestigungselements gering, wenn dieser — wie bevorzugt — aus Tetrafluoräthylen gefertigt ist.

Wenn das zum Befestigen der Elektrodenspitze erforderliche Drehmoment über die Elektrodenzuleitung aufgebracht wird, ist es günstig, die Elektrodenspitze durch eine exzentrisch angeordnete Öffnung der Frontfläche des Hohlzylinders hindurchzuführen, so daß an dieser Stelle die Drehmomentsübertragung stattfinden kann. Die Übertragung kann alternativ auch über — aufeinander abgestimmte — unrunde Querschnitte von Kolben und Hohlzylinder erfolgen. Ein derartiger unrunder Querschnitt wird dabei bevorzugterweise durch einen Stift gebildet, der in einem in Elektrodenlängsrichtung verlaufenden Schlitz gleitet, wobei durch eine Abwinklung bzw. scharfkantig ausgebildete Erweiterung in Umfangsrichtung — bezogen auf die beim Befestigen der Elektrode auftretende Tendenz der Relativbewegung von Kolben und Hohlzylinder — nach Art einer Bajonettführung eine Verriegelung des Befestigungselementes in der ausgefahrenen Stellung bewirkt werden kann, so daß dieses eine definierte Position einnimmt.

Günstig bei der erfindungsgemäßen Lösung ist ferner, daß das Befestigungselement — je nach Anwendungsfall — sowohl stimulierend als auch nicht stimulierend ausgebildet sein kann.

Zur Verdeutlichung sollen die verschiedenen Aspekte der Erfindung — auch soweit sie Gegenstand von Unteransprüchen sind bzw. aus der nachfolgenden Beschreibung im einzelnen hervorgehen — zusammenfassend erläutert werden :

Die Erkenntnis, daß ein an einem dichtend gleitenden Kolben angeordnetes Befestigungselement mit durch Drehbewegung in das Körpergewebe eingreifenden Spitzen vom herzfernen Ende der Elektrodenzuleitung über einfache Handhabungen sowohl aus einer geschützten Stellung ausgefahren werden als auch zum Eingreifen in das Körpergewebe angetrieben werden kann, läßt verschiedene Konzeptionen zu :

1. Hinausschieben des Kolbens über das Befestigungselement durch Druck auf den Mandrins, wenn die gewünschte Fixierungsposition erreicht ist. Anschließend « Einschrauben » des Befestigungselements mit Schraubendreherwirkung des Mandrins. (Durch geeignete Gestaltung der Steigung einer korkenzieherförmigen Befestigungswendel und Leichtgängigkeit der Kolbenführung erfolgt das vollständige Ausfahren des Befestigungselements selbsttätig, wenn dieses in eine Vorbereitungsposition zum Eingriff in das Körpergewebe gebracht wurde.)

2. Um eine Rotation und die damit verbundene Schleuderbewegung des (steifen) Mandrins zu vermeiden, erfolgt das Fixieren des Befestigungselements über ein Drehen der flexiblen Zuleitungswendel, wobei Mittel zur Übertragung des Drehmoments auf den Kolben bzw. das Befestigungselement vorgesehen sind. Erfolgt das Ausfahren der korkenzieherförmigen Befestigungswendel über die beim Eingriff in das Körpergewebe wirksamen Kräfte, so kann die Drehmomentübertragung durch geeignete, diese Relativbewegung gestattende Führungen in Längsrichtung bewirkt werden.

3. Für eine definierte Halteposition der Befestigungsmittel in ausgefahrenem Zustand sorgen bevorzugt eine Bajonettführung bzw. eine geeignete Verengung, die auch als durchgehender Stift ausgeführt sein kann, welche in das durch das schraubenförmige Befestigungselement gebildet Gewinde eingreift und eine Hemmung erzeugt. Diese Hemmung wird vorteilhafter weise in ausgefahrenem Zustand des Befestigungselementes dadurch vergrößert, daß der Wendelabstand in diesem Bereich herabgesetzt ist.

Soll das Befestigungselement stimulierend wirken und dazu die Ausgangsspannung des Herzschrittmachers führen, so wird der das Befestigungselement tragende Kolben bevorzugterweise zum Erzeugen einer galvanischen Verbindung direkt mit der Zuleitungswendel verbunden. Um eine zu starke Beanspruchung der Zuleitungswendel durch eine mögliche Relativdrehung von Kolben und Hohlzylinder zu vermeiden, wird im letzgenannten Fall — wie weiter oben erläutert — ein Momentschluß zwischen diesen beiden Teilen vorgesehen.

Dient zur Stimulation ein Bereich der Frontfläche des Hohlzylinders, so kann es erwünscht sein, die beim Lokalisieren des günstigsten Stimulationspunktes gefundene Potentialschwelle nicht mehr dadurch zu verändern, daß auch die Befestigungsmittel stimulierend wirken. Zu diesem Zweck werden Hohlzylinder und Kolben durch eine isolierende Zwischenschicht potentialmäßig voneinander getrennt.

Wenn das Befestigungselement tangential gerichtete Haken aufweist, die in ausgefahrenem Zustand die Frontfläche des Elektrodenkopfes um nicht mehr als 1 bis 2 mm vorstehen, ist damit die Gefahr einer Myocardperforation durch das in das Gewebe eindringende Befestigungselement auf einfache Weise beseitigt. Durch eine geringe auf die Haken einwirkende Federkraft, die Gewebe zwischen Haken und Frontfläche einklemmt, wird ein fester Kontakt zur stimulierenden Frontfläche der Elektrode aufrechterhalten, so daß die Gewähr gegeben ist, daß die anfänglichen Stimulationsbedingungen für den Zeitraum der Implantation erhalten bleiben.

Weitere vorteilhafte Ausbildungen der Erfindung sind in den Unteransprüchen angegeben. Zwei bevorzugte Ausführungsformen sind in den Figuren vergrößert dargestellt und werden nachfolgend näher beschrieben. Es zeigen :

Figur 1 eine erste Ausführungsform der Erfindung im Längsschnitt,

Figur 2 die Ausführungsform gemäß Fig. 1 in der Draufsicht und

Figur 3 eine andere Ausführungsform der Erfindung im Längsschnitt.

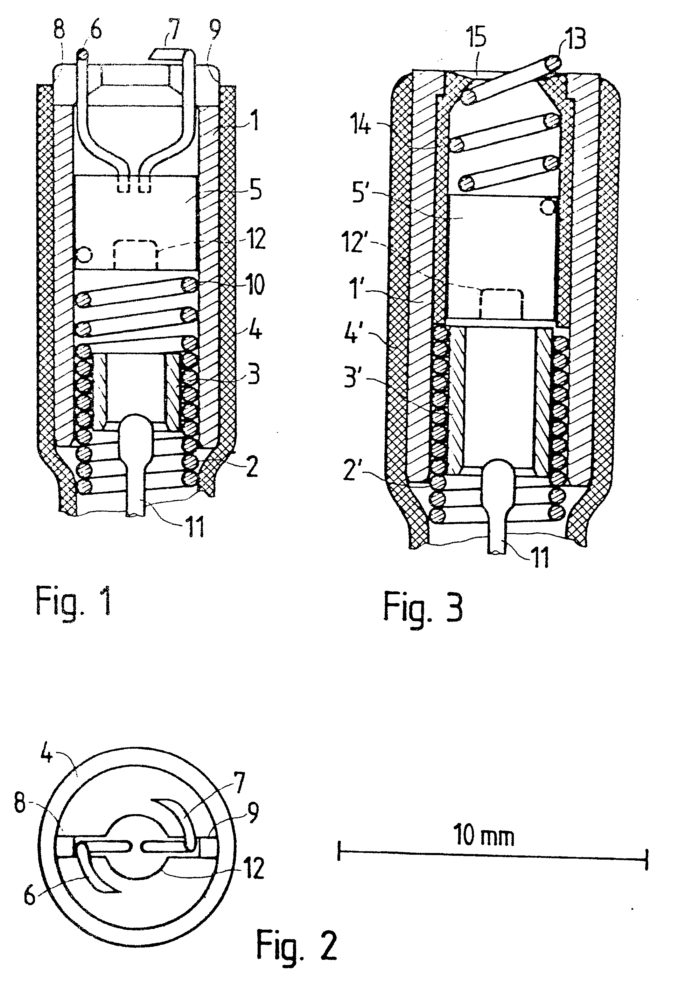

Bei der in Fig. 1 dargestellten Ausführungsform der erfindungsgemäßen Elektrode wird der Elektrodenkopf durch einen Hohlzylinder 1 gebildet, der mit einer die Zuleitung für die Elektrode bildenden Wendel 2 mittels einer Klemmhülse 3 verbunden ist. Der größte Teil des Hohlzylinders 1 und die Zuleitung 2 sind von einem isolierenden Mantel 4 aus flexiblem Silikongummi umgeben. (In den Figuren ist die Zuleitung verkürzt dargestellt und der Stecker zum Verbinden der Elektrode mit dem Herzschrittmacher weggelassen.)

In dem Hohlzylinder 1 ist ein Kolben 5 in Längsrichtung der Elektrode verschiebbar angeordnet. Der Kolben 5 ist in den Hohlzylinder derart dichtend eingepaßt, daß das Eindringen von Körperflüssigkeit vom vorderen (in der Zeichnung oberen) Ende des Hohlzylinders 1 in seinen hinteren Teil verhindert ist. Um die Dichtwirkung durch Erhöhung des lokalen Anpreßdrucks bei gleichzeitiger Verringerung der Reibung zu vergrößern, können den Kolbenumfang umgebende Ringe, Dichtlippen oder dergleichen vorgesehen sein. Der Kolben 5 trägt an seiner vorderen Fläche in zwei Sacklochbohrungen eingefügte Verankerungshaken 6 und 7, die zur Befestigung des Elektrodenkopfes im Herzgewebe dienen. Die Verankerungshaken treten aus der in der Frontfläche des Hohlzylinders 1 vorhandenen Aussparungen 8 und 9 senkrecht aus und sind derart abgewinkelt, daß sie — jeweils einen mit einer Spitze versehen — tangential in die zum Befestigen der Elektrode gewählte Drehrichtung weisen.

In Fig. 1 ist der Kolben in seiner zurückgeschobenen Position zum Einführen der Elektrode in die Vene des Patienten dargestellt. Die Verankerungshaken 6 und 7 liegen an der Frontfläche des Elektrodenkopfes an, so daß sie nicht mit Körpergewebe in Eingriff kommen oder dieses verletzen können. Das hintere Ende des Kolbens 5 ist mit einem freien Ende 10 der Zuleitungswendel verbunden, wobei dieses freie Ende eine größere Steigung aufweist als der jenseits der Klemmhülse 3 gelegene Bereich der Wendel. Es bildet damit eine schraubenfederförmige elektrische Verbindung zwischen Zuleitungs- und Verankerungshaken unter der Voraussetzung, daß auch der Kolben 5 elektrisch leitend ist. Im übrigen steht auch der Hohlzylinder 1 mit der Zuleitung 2 in leitender Verbindung, so daß vor dem Fixieren der Elektrode, d. h. vor dem Ausfahren der Verankerungshaken, bereits eine Schwellenmessung des Herzens vorgenommen werden kann. Die durch das freie Ende der Wendel 10 gebildete Schraubenfeder übt eine geringfügige Zugwirkung auf den Kolben 5 aus, so daß die Verankerungshaken 6 und 7 nicht unbeabsichtigt in ihre ausgefahrene Stellung gelangen können. Diese geringfügige Zugspannung wirkt bei verhakter Elektrode infolge der im wesentlichen waagerecht gerichteten Verankerungshaken durch ein elastisches Nachgeben bei den Herzeigenbewegungen einem Lockern der Elektrode entgegen, wobei die Zugkraft allerdings so klein bemessen sein muß, daß nicht die Gefahr besteht, daß die Verankerungshaken im Laufe der Zeit aus dem Herzgewebe herausgezogen werden. Diese Kraft bewirkt einen verbesserten, gleichbleibenden Kontakt der stimulierenden Elektrodenfrontfläche mit dem Herzgewebe.

Wurde nach dem Einführen der Elektrode in die Herzkammer eine geeignete Position für den Elektrodenkopf gefunden, so werden die Verankerungshaken dadurch ausgefahren, daß durch einen Mandrin 11, der auch zum Führen der (weichen) Elektrodenzuleitung beim Einführen der Elektrode dienen kann und im Inneren der Zuleitungswendel 2 geführt wird, ein Druck auf die Hinterseite des Kolbens 5 ausgeübt wird, der daraufhin im Innern des Hohlzylinders 1 bis zu einem Anschlag vorangleitet, so daß die Verankerungshaken 6 und 7 infolge ihres Abstands von der Frontfläche in das Herzgewebe eingreifen können.

Der Abstand der Haken von der Frontfläche ist so gering gewählt, daß eine Myocardperforation durch ein zu tiefes Eindringen derselben ausgeschlossen ist. Sie beträgt zweckmäßigerweise 1 bis 2 mm.

Bei geeigneter Werkstoffwahl und Dimensionierung des Elektrodenkopfes kann der ausgefahrene Zustand der Verankerungshaken über die Position des Kolbens 5 im Hohlzylinder 1 durch den implantierenden Arzt auf einem Röntgengerät kontrolliert werden.

Die zum Verhaken der Elektrode erforderliche Drehbewegung kann auf zweierlei Weise erzeugt werden : weist der Mandrin 11 ein abgeflachtes Ende und der Kolben 5 eine entsprechend angepaßte Aussparung 12 auf, so kann nach Art eines Schraubendrehers der Kolben und damit die Verankerungshaken 6 und 7 in der tangentialen Richtung ihrer Enden gedreht werden. Obwohl beide Bewegungen vom herzfernen Ende der Zuleitung her über den Mandrin 11 bewirkt werden, kann der Arzt dadurch, daß er einmal eine Schubbewegung und im anderen Fall eine Drehbewegung ausführen muß, schon intuitiv zwischen den beiden Vorgängen unterscheiden.

Bei dem dargestellten Ausführungsbeispiel kann das zum Verhaken der Elektrode notwendige Drehmoment auch über die Wendel selbst aufgebracht werden, was den Vorteil hat, daß der Führungsmandrin in diesem Fall nicht gedreht zu werden braucht und damit infolge seines abgewinkelten Verlaufs keine Veränderung der Lage des Elektrodenkopfes beim Verhaken eintreten kann. In diesem Fall braucht der Mandrin 11 kein abgeflachtes Ende aufzuweisen und auch auf die Aussparung 12 im Kolben 5 kann verzichtet werden. Die Übertragung des Moments vom Hohlzylinder 1 auf die Verankerungshaken 6 und 7 erfolgt vielmehr durch eine entsprechende auf die Haken 6 und 7 einwirkende Aussparung 12 in der Stirnfläche des den Elektrodenkopf bildenden Hohlzylinders 1, wie es in Fig. 2 in der Draufsicht erkennbar ist.

Die Übertragung des zum Verhaken der Elektrode erforderlichen Drehmoments vom Hohlzylinder 1 auf den Kolben 5 erfolgt bei einer weiteren — nicht dargestellten — Ausführungsform der Erfindung durch eine oder mehrere in Längsrichtung verlaufender Führungen, in die geeignete, entsprechend angepaßte Führungsstifte eingreifen. Dabei ist es unwesentlich, welches dieser Elemente am Kolben und welches am Hohlzylinder angebracht ist. Es muß lediglich dafür Sorge getragen werden, daß die Schlitze nicht soweit durchgehend ausgebildet ist, daß durch sie hindurch Körperflüssigkeit am Kolben 5 vorbei in das Innere der Zuleitungswendel 2 gelangen kann. Ist der Verlauf der Schlitze nicht gradlinig, sondern abgewinkelt oder erweitert er sich an einer Stelle zu einem größeren Bereich, so ergibt sich eine Sicherung des Kolbens 5 gegen Zurückgleiten, sobald der Kolben zum Einhaken der Verankerungshaken 6 und 7 gedreht wird und somit in den erweiterten Bereich gelangt. Die Wirkung der Anordnung entspricht dabei derjenigen der für Glühlampen bekannten Bajonettfassungen, wobei der Kolben 5 dem Lampensockel gleichzusetzen ist. Die Richtung der Abwicklungen des Führungsschlitzes hängt davon ab, ob das Drehmoment zum Verankern der Elektrode direkt über den Kolben oder über die Zuleitungswendel aufgebracht wird, da damit entweder der Hohlzylinder 1 gegenüber dem Kolben 5 antreibend ist — sich also gegenüber diesem voranbewegt während er im anderen Fall vom Kolben angetrieben wird und die Tendenz hat, ihm gegenüber zurückzubleiben.

Bei der in Fig. 3 dargestellten Ausführungsform der erfindungsgemäßen Elektrode entsprechen diejenigen Bauelemente, deren Bezugszeichen mit einem « ' » versehen ist, in ihrer Funktion im wesentlichen den äquivalenten Elementen der in den Fig. 1 und 2 wiedergegebenen Ausführungsformen, wenn auch bestimmte — den im folgenden beschriebenen Funktionen dienende Modifikationen vorgenommen wurden.

Als Befestigungselement dient bei dieser Ausführungsform eine korkenzieherartige, schraubenförmige Wendel 13, die mit dem Kolben 5' fest verbunden ist und über den in die rückwärtige Aussparung 12' des Kolbens 5' eingreifenden Mandrin 11' in das Herzgewebe einschraubbar ist. Vorteilhaft bei dieser Ausführung ist, zum ein Hinausschieben der schraubenförmigen Wendel 13 am vorgesehenen Befestigungspunkt des Elektrodenkopfes keine der Druckkraft des Führungswerkzeugs (Mandrin 11') entgegenwirkende Zugkraft über die Zuleitungswendel erzeugt werden muß. Der erforderliche Gegendruck kann vielmehr durch die Gewebeoberfläche aufgebracht werden, an der die Stirnfläche des Hohlzylinders 1' anliegt. Dabei wird die Wendel zunächst nicht vollständig ausgefahren, sondern tritt nur soweit aus dem Elektrodenkopf aus, wie es notwendig ist, damit sie ins Herzgewebe eindrehbar ist. Bewegt der Arzt, nachdem er beim Voranschieben des Mandrins 11 einen Widerstand spürte, diesen nach Art eines Schraubendrehers, so wird die Wendel 13 in das Herzgewebe hineingeschraubt, wobei sich der Kolben 5' infolge der Steigung der Wendel 13 und unterstützt durch den vom Mandrin 9 ausgeübten Druck bis zu einem Anschlag voranbewegt.

Die für das beim Einschrauben erforderliche Voranschieben der Wendel 13 samt Kolben 5' notwendige Kraft wird hierbei nur durch die Schraubenwirkung der Wendel in bezug auf das Gewebe bzw. eine Druckwirkung auf den Kolben 5' über den Mandrin 11' erzeugt. Weisen der Kolben 5' und der Hohlzylinder 1' in gegenseitiger Anpassung in der Zeichnung nicht dargestellte — in Längsrtichtung angeordnete Führungsschlitze und -stifte auf, so kann das Einschraubmoment für die Wendel 13 (entsprechend dem in Figuren 1 und 2 dargestellten Ausführungsbeispiel) auch über die Zuleitungswendel 2' bei festgehaltenem Mandrin 11 aufgebracht werden.

Wie auch bei dem zuvor beschriebenen Ausführungsbeispiel kann die Elektrode ohne weiteres wieder aus ihrer Verankerung gelöst werden, wenn der Mandrin in die entgegengesetzte Richtung gedreht und nach dem Lösen des Elektrodenkopfes an der Zuleitungswendel gezogen wird. Die Elektrode läßt sich für eine erneute Einführung vollständig herausziehen, ohne daß eine Gewerbeverletzung zu befürchten ist. Um die Elektrode für einen weiteren Einführungsvorgang vorzubereiten, braucht der Kolben 5 lediglich durch eine einfache Handhabung in den Hohlzylinder zurückgeschoben werden, so daß die Verankerungsmittel wieder ihre geschützte Position einnehmen. Bei der Ausführungsform gemäß Fig. 3 ist der Kolben 5' und damit die Wendel 13, von dem Hohlzylinder 1' durch eine Isolierhülse 14 aus Teflon (eingetragenes Warenzeichen) potentialmäßig getrennt. Die Stimulationsimpulse werden daher ausschließlich über die Frontfläche des Hohlzylinders 1 dem Gewebe mitgeteilt, wobei die Wendel 13 die Aufgabe der Fixierung hat. Damit besteht die Gewähr, daß sich die Werte des Schwellenpotentials durch das Befestigen der Elektrode nicht mehr verändern, d. h. ein einmal gefundener günstiger Wert beibehalten wird.

Eine Verengung 15 des Innenquerschnitts der Austrittskante der Isolierhülse 14 bildet einerseits einen Anschlag für den Kolben 5' sowie eine zusätzliche Dichtung und stellt andererseits durch Eingriff in die Wendel 13 eine Hemmung gegen ein ungewolltes Zurückgleiten der ausgefahrenen Wendel 13 in den Hohlzylinder 1' im Verlauf der Applikationszeit aufgrund der Herzbewegungen dar. Die Verengung 15 erzeugt (bei einer alternativen Ausführung auch in Form eines quer in der Öffnung angeordneten, durchgehenden Stiftes) in Bezug auf die Steigung der Wendel 13 die Wirkung eines Gewindes und ist somit in der Lage das Ausfahren des Befestigungselements zumindest zu unterstützen. Eine Verringerung der Steigung der Wendel in Richtung zum herzfernen Ende der Elektrode hin ermöglicht durch die damit erzeugte Klemmwirkung zusätzlich ein Zurückbewegen der Wendel in der ausgefahrenen Position.

## Ansprüche

1. Elektrode für einen künstlichen Herzschrittmacher für transvenöse Anwendung mit mindestens einem Befestigungselement (6, 7, 13), das an einem in einem mit einer Zuleitungswendel (2) verbundenen Hohlzylinder (1) geführten Kolben (5) derart angeordnet ist, daß es zum Einführen der Elektrode soweit in den Hohlzylinder hineinschiebbar ist, daß es nicht mit dem Herzgewebe in Eingriff kommt, während es zum Fixieren der Elektrode durch eine mittels eines sich innerhalb der Zuleitungswendel erstreckenden Führungswerkzeugs (11) ausgeübte Kraft über deren Frontfläche hinaus herausschiebbar ist, dadurch gekennzeichnet, daß das Befestigungselement (6, 7, 13) mindestens ein im wesentlichen tangential gerichtetes angespitztes Ende aufweist, welches durch ein auf das Befestigungselement ausgeübtes Drehmoment mit dem Herzgewebe in Eingriff bringbar ist, daß zum Ausüben des Drehmoments über das Führungswerkzeug (11) unter Einschluß des Kolbens (5, 5') oder über die Zuleitungswendel, am rückwärtigen Ende des Kolbens mit dem Führungswerkzeug drehmomentschlüssig in Eingriff bringbare Kupplungsmittel (12, 12') bzw. Mittel (8, 9) zum Übertragen des Drehmoments von dem mit der Zuleitungswendel verbundenden Hohlzylinder (1, 1') auf das Befestigungselement vorgesehen sind, und daß der an der Innenfläche des Hohlzylinders (1, 1') anliegende Kolben mindestens im ausgefahrenen Zustand des Befestigungselements eine Dichtung gegen in das Innere der Zuleitungswendel eindringende Körperflüssigkeit bildet.

2. Elektrode nach Anspruch 1, dadurch gekennzeichnet, daß die zum Herausschieben des schraubenförmig gewendelten Befestigungselements aus dem Hohlzylinder erforderliche Kraft durch die Gewindewirkung des gewendelten Befestigungselements in Bezug auf eine an dem Hohlzylinder angeordnete, mindestens in Teilen vorhandene, Verengung und/oder durch die Gewindewirkung des beim Fixieren der Elektrode in das Körpergewebe eindringenden gewendelten Befestigungselements erzeugbar ist.

3. Elektrode nach Anspruch 2, dadurch gekennzeichnet, daß die Steigung der Wendel des Befestigungselements in Richtung auf das eingespannte Ende hin verringert ist.

4. Elektrode nach einem der vorangehenden Ansprüche, dadurch gekennzeichnet, daß zum Ausüben des Drehmoments über die Zuleitungswendel in dem frontseitig abgeschlossenen Hohlzylinder mindestens eine exzentrisch gelegene Durchtrittsöffnung (8, 9) für das Befestigungselement (6, 7) vorgesehen ist.

5. Elektrode nach einem der Ansprüche 1 bis 3, dadurch gekennzeichnet, daß zur Drehmomentsübertragung zwischen Hohlzylinder (1, 1') und Kolben (5, 5') an diesen mindestens ein Paar aneinander angepaßter Mitnehmerelemente, bestehend aus Führungsstift und, im wesentlichen in Längsrichtung verlaufendem, -schlitz vorgesehen sind.

6. Elektrode nach Anspruch 5, dadurch gekennzeichnet, daß Schlitz und Führungsnocken in der ausgefahrenen Stellung des Befestigungselements eine Bajonettverriegelung bilden.

7. Elektrode nach einem der vorangehenden Ansprüche, dadurch gekennzeichnet, daß Kolben (5) und Zuleitungswendel (2) galvanisch und/oder mechanisch miteinander verbunden sind.

8. Elektrode nach einem der Ansprüche 1 bis 6, dadurch gekennzeichnet, daß Kolben (5') und Befestigungselement (13) einerseits sowie Hohlzylinder (1') und Zuleitungswendel (2') andererseits durch eine Zwischenschicht (14) voneinander elektrisch isoliert sind.

9. Elektrode nach einem der vorangehenden Ansprüche, dadurch gekennzeichnet, daß das Befestigungselement aus mindestens zwei Haken (6, 7) mit tangential in Drehrichtung, bezogen auf das Fixieren der Elektrode, gerichteten Spitzen besteht.

10. Elektrode nach Anspruch 9, dadurch gekennzeichnet, daß die Haken (6, 7) in der Position zum Eingreifen in das Gewebe die Frontfläche des Elektrodenkopfes um ungefähr 1 bis 2 mm überragen.

## Claims

1. Electrode for an artificial heart pace-maker for transvenous use with at least one attaching element (6, 7, 13), which is arranged on a plunger (5), which is guided in a hollow cylinder (1) connected to a lead-in coil (2), so that for the insertion of the electrode the attaching element may be slid so far into the hollow cylinder that it does not come into contact with the cardiac tissue, and for fixing the electrode the attaching element may be slid out beyond the front surface there of by a force exerted by a guide tool (11) which extends within the lead-in coil, characterised in that, the attaching element (6, 7, 13) has at least one pointed end directed substantially tangentially, which may be brought into engagement with the cardiac tissue by a twist exerted on the attaching element, that, for exerting the twist *via* the guide tool (11) with the inclusion of the plunger (5, 5') or *via* the lead-in coil, there are provided at the rear end of the plunger coupling means (12, 12') which can be brought into turning contact with the guide tool and/or means (8, 9) for the transmission of the twist from the hollow cylinder (1, 1') connected to the lead-in coil to the attaching element, and that the plunger lying against the interior surface of the hollow cylinder (1, 1') forms a seal against body fluid leaking into the interior of the lead-in coil, at least in the extended position of the attaching element.

2. Electrode according to claim 1, characterised in that the force required to slide the helically coiled attaching element out of the hollow cylinder can be produced by the screw effect of the coiled attaching element relative to a constriction, provided at least in parts, arranged on the hollow cylinder and/or by the screw effect of the attaching element coiled upon fixing the electrode and penetrating the body tissue.

3. Electrode according to claim 2, characterised in that the pitch of the coil of the attaching element is reduced towards the fixed end.

4. Electrode according to any one of the preceding claims, characterised in that, for the exertion of the twist *via* the lead-in coil there is provided in the hollow cylinder closed at the front, at least one eccentrically situated opening (8, 9) for the attaching element (6, 7).

5. Electrode according to any one of claims 1 to 3, characterised in that, for the transmission of twist between hollow cylinder (1, 1') and plunger (5, 5'), they are provided with at least one pair of mutually fitting cam elements consisting of a guide pin and a guide slot extending substantially longitudinally.

6. Electrode according to claim 5, characterised in that the slot and guide cam form a bayonet locking mechanism in the extended position of the attaching element.

7. Electrode according to any one of the preceding claims, characterised in that plunger (5) and lead-in coil (2) are electrically and/or mechanically interconnected.

8. Electrode according to any one of claims 1 to 6, characterised in that plunger (5') and attaching element (13) on the one hand and hollow cylinder (1') and lead-in coil (2') on the other hand are electrically insulated from each other by an intermediate layer (14).

9. Electrode according to any one of the preceding claims, characterised in that the attaching element consists of at least two hooks (6, 7) with tips directed tangentially in the direction of twisting during fixing of the electrode.

10. Electrode according to claim 9, characterised in that the hooks (6, 7) project above the front surface of the electrode top by about 1 to 2 mm in the position for engagement in the tissue.

## Revendications

1. Electrode pour stimulateur cardiaque artificiel, implantée par voie veineuse et comprenant au moins un élément de fixation (6, 7, 13) qui est disposé dans un piston (5), guidé dans un cylindre creux (1) relié à une hélice d'alimentation (2), de façon à pénétrer par translation dans le cylindre creux, pour l'introduction de l'électrode, sans venir en contact avec le tissu cardiaque, mais que la force exercée par un outil de guidage (11), logé à l'intérieur de l'hélice d'alimentation, repousse au-delà de sa face frontale pour la fixation de l'électrode, cette dernière étant caractérisée en ce que l'élément de fixation (6, 7, 13) présente au moins une extrémité appointée sensiblement tangentielle, qu'un couple exercé sur l'élément de fixation fait pénétrer dans le tissu cardiaque ; l'extrémité arrière du piston porte des moyens d'accouplement (12, 12') qu'un couple fait engrener avec l'outil de guidage (11) pour exercer le couple par l'intermédiaire dudit outil (11) et du piston (5, 5') ou par l'hélice d'alimentation, ou des moyens (8, 9) sont prévus sur l'élément de fixation pour transmission du couple du cylindre creux (1, 1') relié à l'hélice d'alimentation ; et le piston appliqué sur la face intérieure du cylindre creux (1, 1') constitue, au moins dans l'état sorti de l'élément de fixation, un joint d'étanchéité à l'humeur pénétrant à l'intérieur de l'hélice d'alimentation.

2. Electrode selon revendication 1, caractérisée en ce que la force nécessaire pour faire sortir l'élément de fixation hélicoïdal du cylindre creux peut être produite par l'effet de filetage de l'élément de fixation hélicoïdal sur un rétrécissement prévu sur le cylindre creux, au moins sur certaines parties, et/ou l'effet de filetage de l'élément de fixation hélicoïdal pénétrant dans le tissu lors de la fixation de l'électrode.

3. Electrode selon revendication 2, caractérisée par une diminution du pas de l'hélice de l'élément de fixation vers l'extrémité encastrée.

4. Electrode selon une quelconque des revendications 1 à 3, caractérisée par au moins une ouverture excentrique (8, 9) sur la face frontale fermée du cylindre creux pour le passage de l'élément de fixation (6, 7), afin d'exercer le couple par l'intermédiaire de l'hélice d'alimentation.

5. Electrode selon une quelconque des revendications 1 à 3, caractérisée en ce que pour la transmission du couple entre le cylindre creux (1n 1') et le piston (5, 5') ces derniers portent au moins une paire d'éléments d'entraînement conjugués, constitués par une broche de guidage et une fente de guidage sensiblement longitudinale.

6. Electrode selon revendication 5, caractérisée en ce que la fente et la broche de guidage constituent un verrouillage à baïonnette dans la position sortie de l'élément de fixation.

7. Electrode selon une quelconque des revendications 1 à 6, caractérisée en ce que le piston (5) et l'hélice d'alimentation (2) sont reliés galvaniquement et/ou mécaniquement.

8. Electrode selon une quelconque des revendications 1 à 6, caractérisée en ce que le piston (5') et l'élément de fixation (13) d'une part, et le cylindre creux (1') et l'hélice d'alimentation (2') d'autre part sont isolés électriquement par une couche intermédiaire (14).

9. Electrode selon une quelconque des revendications 1 à 8, caractérisée en ce que l'élément de fixation est constitué par au moins deux crochets (6, 7) munis de pointes tangentielles dans le sens de rotation, par rapport à la fixation de l'électrode.

10. Electrode selon revendication 9, caractérisée en ce que les crochets (6, 7) sont en saillie d'environ 1 à 2 mm sur la face frontale de la tête d'électrode, dans la position de pénétration dans le tissu.

Fig. 1

Fig. 3

Fig. 2

10 mm